(19) Europäisches Patentamt / European Patent Office / Office européen des brevets

(11) **EP 4 430 959 A2**

(12) **EUROPEAN PATENT APPLICATION**

(43) Date of publication:
**18.09.2024 Bulletin 2024/38**

(21) Application number: **24192131.1**

(22) Date of filing: **05.10.2020**

(51) International Patent Classification (IPC):
**A23L 33/125** (2016.01)

(52) Cooperative Patent Classification (CPC):
(C-Sets available)
**A61P 1/12; A23K 10/30; A23K 10/37;
A23K 20/163; A23K 50/40; A23L 33/105;
A23L 33/125; A23L 33/24; A61K 31/702;
A61K 36/185; A61K 36/28; A61P 3/04;
A61P 31/04;** A23V 2002/00; Y02P 60/87    (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.10.2019 GB 201914384**

(62) Document number(s) of the earlier application(s) in
accordance with Art. 76 EPC:
**20804016.2 / 4 037 692**

(71) Applicant: **MARS INCORPORATED
McLean, VA 22101 (US)**

(72) Inventors:
• **MARSHALL-JONES, Zoe
Leicestershire, LE14 4RT (GB)**

• **WATSON, Phillip
Leicestershire, LE14 4RT (GB)**
• **LE BON, Melanie
Leicestershire, LE14 4RT (GB)**

(74) Representative: **Haseltine Lake Kempner LLP
One Portwall Square
Portwall Lane
Bristol BS1 6BH (GB)**

Remarks:
This application was filed on 31-07-2024 as a
divisional application to the application mentioned
under INID code 62.

(54) **MICROBIOME INTERVENTIONS**

(57) The present disclosure relates to compositions comprising a range of ingredients suitable for use in adjusting and/or treating a companion animal such as a canid (e.g. a dog) or a feline (e.g. a cat) and their microbiomes, monitoring tools, and diagnostic methods for determining the health of a companion animal and their microbiome.

FIG.1

**EP 4 430 959 A2**

(52) Cooperative Patent Classification (CPC): (Cont.)

C-Sets
**A61K 31/702, A61K 2300/00;**
**A61K 36/185, A61K 2300/00;**
**A61K 36/28, A61K 2300/00;**
A23V 2002/00, A23V 2200/3202, A23V 2250/284,
A23V 2250/5108

**Description**

**TECHNICAL FIELD**

The present disclosure relates to compositions for use in adjusting and/or treating companion animals and their microbiomes, monitoring tools, and diagnostic methods for determining the health of a companion animal and their microbiome.

**BACKGROUND TO THE INVENTION**

The microbiome is described as all of the microorganisms in any particular environment and is more specifically the combined genetic material of the microorganisms in that environment. In mammals the microbes exist in a symbiotic relationship with their host being present on the skin, in the gut and in the oral cavity and indeed these microorganisms play an important role in the host's health forming a barrier to colonisation with foreign microbes and hence protecting the animal against pathogens as well as in the gut aiding the breakdown of nutrients releasing energy and producing vitamins essential to life.

Although the influence of pathogenic and probiotic microbes on their mammalian hosts have long been appreciated there is now a growing appreciation of the influence of the total community composition the detailed microbial balance and the genetic potential of the inhabiting microbiota. Non-invasive studies of the gut microbiome in humans are predominantly conducted using a faecal surrogate samples that are particularly representative of the distal colon, the major site of bacterial growth within the intestinal tract. Bacterial growth in the colon occurs through fermentation, a process using dietary ingredients that remain undigested by the host and hence are not absorbed being left available for influencing the gut microbial communities and the microbiome. Thus, an equilibrium state is reached whereby in the absence of gross changes in diet or medication or incoming infective agents, the predominating microbial community types are relatively stable within individual animals over time.

Development of the microbiome occurs shortly following birth in mammals, though a combination of maternal or parental and environmental inoculation and the interplay or bacteria with the immune system is considered to play a role in immune priming for future recognition of pathogenic microbes and intestinal physiology. Based on studies in human infants these early developments in the gut microbiome are now considered to impact health throughout later life In the weeks and months following birth, a rapid increase in diversity occurs representing the early establishment of gut microbiome development during this stage the microbiome and microbial communities existing are considered to be plastic or malleable. In part as a result of this pre-diversity development stage in the early life stages, puppies have an under-developed gut barrier, which includes the gastrointestinal microbiome as well as histological and gut associated immune functions. Puppies and young dogs are therefore are more prone to gastrointestinal illnesses such as soft malformed faeces, diarrhoea, flatulence and sickness. The increase in diversity during maturation leads to the development of an adult gut microbiome that can be resilient to colonization even by beneficial microbes such as probiotic species and hence the adult microbiome is relatively resilient resisting large shifts in community structure and intestinal dysbiosis. The microbiome of adult animals contains similar bacterial communities but is more diverse and well developed with an adjusted community structure representing a more robust and resilient microbiota with a gut microbiome showing enhanced resilience compared to other life stages.

Senior and geriatric dogs and people are also more prone to digestive issues such as diarrhoea and gastrointestinal infections which can have heightened severity and greater implications. These gastrointestinal issues may occur in part as a result of a deterioration in the gut microbiome.

In adult dogs altered gut communities may also occur by drift over time or through larger scale shifts in composition due to environmental and diet factors. Infectious agents entering the digestive system or altered nutrients available to the microbial community due to diet changes can disrupt the balance of the microbiome leading to dysbiosis. Dysbiosis is described as an unbalancing of the microbial communities and, in the gut, can lead to clinical signs including gastrointestinal upset, diarrhoea, vomiting, nutritional deficiency and weight loss. Although the microbiome and microorganisms that exist within the gut are present in a continuum of abundance from shortly after birth or weaning until the late geriatric life stages, an assault to the microbiome such as caused by infection, antibiotic clearance, medication or extreme diet change can alter the community composition throughout life. In some cases, long-lasting effects on the health of the host such as chronic diarrhoea, lactose in tolerance. In cases where dogs have recurrent or chronic diarrhoea a characteristic pattern, signature or fingerprint in the microbiome may be detected and may indicate the likelihood of treatment being effective through dietary change and consequent changes in the microbiota.

The effect of nutrition on the microbiome is particularly suited to research in dogs and cats since complete and balanced diets may be fed as the sole source of food for extended periods of time, thus reducing confounding effects of dietary preference between individuals. That said, despite decades of research towards improving gastrointestinal (GI) health and optimizing faeces quality, there remains a very limited understanding of the bacterial taxa and functional gene groups associated with health parameters such as faecal form, apparent GI health (lack of clinical symptoms, resilience to challenge and recovery from infection) as well as GI dysfunction. This is particularly evident in hosts beyond the human such as dogs and cats.

Many ingredients are reported to alter host health and functional foods such as prebiotics and fibre are of particular use for gastrointestinal health and faeces quality or faecal consistency. In particular complex mixtures of soluble and insoluble fibre, prebiotic oligosaccharides, glycans or beta-glucans can represent long recognised functional dietary ingredients that can be helpful for rectifying gut health conditions such as chronic and acute diarrhoea or bloating of the digestive system improving the health wellness and vitality of the host.

Studies assessing the effect of dietary fibre are particularly numerous since microbial populations in the large intestine selectively ferment digestion resistant fibre. As a result, there is a large body of data available from in vitro and in vivo testing of specific food ingredients including soluble and insoluble fibres and prebiotics. Such ingredients are also known to effect microbial populations within the intestinal tract and in particular within the colon differentially effecting populations that enhance health through fermentation of resistant fibre not digested by the host. Research to assess the effect of dietary fibre on the microbiota including soluble fibre, prebiotics and insoluble complex resistant dietary fibre considered important for bulking properties and intestinal transit are numerous and have demonstrated effects both on faeces consistency and the microbial or microbially derived content of faeces.

Research findings can be difficult to compare in part due to the variation and complexity of many ingredients and fibre sources hence uncovering consistent trends remains problematic. Most naturally occurring dietary fibre sources represent mixtures of soluble and insoluble fibre with substantial research effort focusing on prebiotic oligosaccharides, glycans and beta-glucans. Specific product, inclusion level and host species are also confounding factors in the assessment of trends, however these ingredients have demonstrated effects both on faeces consistency and the microbial or microbially derived content of faeces (Ferrario et al., 2017; Wakshlag et al., 2011; Simpson et al., 2001; Sunvold et al.. 1995; Vickers et al., 2001). Although dosage, as well as source specific characteristics such as intake level are key to effectiveness, meta-analyses of human research studies on fibre and gastrointestinal health uncovered a relationship between fibre intake and incidence of colorectal cancer. High fibre intake although product / ingredient and inclusion level specific was described to reduce the risk of colorectal cancer in particular consumption of whole grains and cereal-derived fibres were reported to be potentially protective.

The research literature describing fibre and prebiotic dietary supplementation in pet animals to date largely defines bacterial changes associated with particular ingredients measured by selective bacterial culture or now out-dated molecular techniques (such as denaturing gradient gel electrophoresis) often measuring those taxa known to be beneficial to human health. Whether these insights are valid across different hosts and are therefore relevant to canine health remains unclear. However, a small number of studies have been published that address changes in the microbiota as measured by deep sequencing upon feeding specific fibre sources and prcbiotics in dogs. Middlebos et al., (2010) fed a diet containing 7.5% sugar beet pulp to dogs and found this amount to alter the structure of the gut microbiota detectably compared to the reference diet. Although the data supported only phylum level changes, significant increases in the Firmicutes and reductions in the Fusobacteria were observed when dogs received the diet containing sugar beet pulp.

In the manipulation of the faecal microbiota towards the enhancement of health, an understanding of the long term effects of dietary influence on the microbiome and the impact on host health parameters such as clinical biomarkers of health and gastrointestinal resilience and recovery would represent a significant advancement to this area of research.

Faeces consistency and particularly the extremes of loose or dry faeces are key indicators to owners of pet health and abhorrent faeces quality, diarrhoeic episodes and poor gastrointestinal health that lead to hygiene and lifestyle inconveniences are reportedly a major blocker to pet ownership, can have a significant effect on human animal interactions and hence can cause quality of life issues for people and their pet animals. As such, the impact of diet and ingredients on faeces quality in dogs and cats is of interest for the optimisation of pet health, wellness, lifestyle, vitality and nutrition. Dietary intake, including both dry matter volume, moisture and nutrient content can impact faeces consistency.

However, research on the gut microbiome and links with animal health is limited. Thus, there is a need for understanding the relationship between the gut microbiome and animal health wellness and vitality. Measuring, monitoring and tracking of gut bacteria in faeces over time or with nutritional intake or intervention to enhance or support a healthy gut in pets would additionally be advantageous since this enable the pet owner to observe the effect of the composition, ingredient or nutritional factor and provides the impetuous for the pet owner to continue providing the health enhancing factor. Furthermore, there is a need for novel methods and compositions for enhancing gastrointestinal resilience, gut health in healthy pets and also for treating both chronic and acute intestinal disorders such as acute self-limiting diarrhoeal infections and chronic enteropathies that are associated with the microbiome Furthermore, there is a need to support gastrointestinal resilience following veterinary treatments and medicaments that effect the microbiome such as compositions ingredients and dietary boosters or toppers that reduce the occurrence of microbiome dysbiosis and the resulting diarrhoea or soft faeces. Compositions or ingredients with such a utility would provide additional benefit aiding veterinarians in managing gastrointestinal symptoms of veterinary treatment.

Given the importance of the microbiome to health and wellbeing it is important to find ways to influence and to monitor and the status of the microbiome of an animal and to enable owners to observe the benefit through tracking health status over time, because of the inherent changes in the gut barrier, resilience to diarrhoea and gastrointestinal health that can occur with altered microbiome contents based on nutrient intake provided by owners and which impact on the health wellness and vitality of the pet.

## SUMMARY OF THE INVENTION

The present disclosure relates to compositions that change the microbiome of a companion animal such as a canid and methods comprising administering and using such compositions.

The compositions and methods influence, optimise and enhance a companion animal's gut microbiome and impacts gastrointestinal health and resilience and therein improving the health, wellness and vitality of the animal. Additionally, the present disclosure relates to methods to monitor the microbiome through single and multi-point testing methods to enable the influence of the composition to be tested for and effect on the microbiome and as such to generate microbiome based gut health and resilience care pathways for enhancing pet wellness. The methods of the present disclosure can achieve this with high accuracy, as shown in the examples.

In certain embodiments, the disclosure provides a composition such as, a pet food such as a nutritionally complete pet food such as a dry (e.g., kibbles), semi-moist or moist pet food), a non-nutritionally complete pet food such as a supplement, functional topper, functional food booster, or nutraceutical or pharmaceutical composition for companion animals containing a combination of at least one of the ingredients selected from: galactooligosaccharides (GOS) of about 0.05 g/day to about 5.0 g/day; beet pulp and/or chicory pulp to a total amount of about 0.1 g/day to about 10 g/day; and cellulose from about 0.2 g/day to about 30.8 g/day.

In certain embodiments, the product includes at least two of the ingredients, preferably at least three of the ingredients in combination at their minimum level. In certain embodiments, the ingredients in combination is at a concentration up to the maximum levels as described above.

In certain embodiments, the composition further comprises an additional prebiotic. In certain embodiments, the composition further comprises an additional fibre or other functional food ingredient.

In certain embodiments, the composition further contains a probiotic species of lactic acid bacterium such as a *Bifidobacterium, Lactobacillus* or *Enterococcus.* In certain embodiments, composition further contains a probiotic species of yeast such as a species from the genus *Saccharomyces.* In certain embodiments, the composition further contains a spore forming probiotic bacterial species such as a species from the genus *Bacillus.*

In certain embodiments, the composition improves intestinal health in a companion animal within about 3 - 21 days after administering the composition to the companion animal. In certain embodiments, the composition is a dietary supplement, also known as a booster. In certain embodiments, the dietary supplement is added to the top of the pet food as a topper. In certain embodiments, the dietary supplement is subsequently mixed throughout the product. In certain embodiments, the composition is a dog food product.

In some embodiments, the presently disclosed subject matter provides a method of improving intestinal health and resilience in a healthy companion animal or in an animal in need of improved gastrointestinal robustness such as an animal suffering acute or recurrent diarrhoea thereby improving resilience, health and wellness. In certain embodiments, the method comprises administering to the companion animal an effective amount of any composition disclosed herein.

Also provided is a method of determining the health of a companion animal's microbiome, comprising quantitating one, two, three, four or more bacterial taxa (e.g., certain families or genera or species) to determine their abundance; and comparing the abundance to the abundance of the same bacterial taxa in a control data set: wherein an increase or decrease in the abundance of the four or more bacterial taxa relative to the control data set is indicative of the health status of the microbiome. A healthy microbiome can be associated with reduced pathogen load, and short chain fatty acid production. A reduced pH in the gut lumen is associated with reduced permeability of the gut barrier and gastrointestinal resilience. An unhealthy microbiome with pathogenic microorganisms represented at a higher bacterial load is associated with a number of health conditions. It is therefore desirable to monitor the health of the gut microbiome or to diagnose an unhealthy microbiome.

Further provided is a method of determining the health of a companion animal's microbiome, comprising detecting at least one. two, preferably at least three, preferably at least four bacterial taxa in a sample obtained from the companion animal; wherein the presence of the at least four bacterial taxa is indicative of an unhealthy microbiome.

The health of a companion animal's microbiome may also be determined by a method comprising the steps of calculating the diversity index for the species within the companion animal's microbiome and comparing the diversity index to the diversity index of a control data set.

The invention also provides a method of monitoring a companion animal such as a canid, comprising a step of determining the health of the companion animal's microbiome by a method of the invention on at least two time points.

In one embodiment, the health may be determined through comparison to physical characteristics such as faeces form, stool consistency and/or gastrointestinal resilience or in relation to weight maintenance through stabilisation of the microbiome. This is particularly useful where a companion animal is receiving treatment to shift the microbiome as it can monitor the progress of the therapy. It is also useful for monitoring the health of the companion animal.

Also provided is a method of monitoring the health of the microbiome in a companion animal who has who has received the composition of the present invention which is able to change the microbiome composition, comprising determining the health of the microbiome by a method according to the present disclosure. Such methods allow a skilled person to determine the success of the treatment. Preferably these methods comprise determining the health of the microbiome before and after treatment as this helps to evaluate the success of the treatment. These approaches also aid the ability also predict the likely success or outcome for other pets presenting with similar symptoms (i.e., to inform prognostic approaches). The presently disclosed subject matter hereby provides a method by which health may be enhanced with receipt of a composition of dietary ingredients through a main meal or complementary pet care or pet food product in combination with methods for the determination of the gastrointestinal health of the animal, such that the owner or attending veterinarian is able to observe the impact on gastrointestinal health and resilience and thus is able to observe an effect of feeding a composition on the animal and determine whether the animal will or has benefitted from an intervention to bring the microbiome back to its healthy state depending on the timing of the testing compared to feeding of the composition or pet food product.

The presently disclosed subject matter additionally provides a method for assessing the intestinal health status in a healthy companion animal without signs of gastrointestinal upset and determine whether the canid will benefit from an intervention to bring the microbiome back to its healthy state. In certain embodiments, the presently disclosed subject matter provides a method for determining the intestinal health status in a companion animal in need thereof such as an animal with clinical signs such as diarrhoea or poor faeces quality or with intestinal dysbiosis such as chronic enteropathy or TBD.

In certain embodiments, the presently disclosed subject matter provides a method for determining the intestinal health status in a companion animal prior to and/or after receiving composition the composition of the present invention thereby assessing the need for receiving the pet care product. The intestinal health status, for example, can be measured by using faeces scoring system such that the Waltham faeces scoring system. https://www.waltham.com/sites/g/files/jydpyr1046/files/2020-05/waltham-scoring.pdf

In certain embodiments, the presently disclosed subject matter provides a method for assessing the intestinal health status in a companion animal before, during and after receiving a pet care product such as a composition, such as a supplement a pet food functional topper or booster or a nutritionally complete dry kibble food thereby determining and monitoring gastrointestinal health of the animal before during and after receipt of the product such that the success of the pet care product can be assessed.

In certain embodiments, the amount of a bacterial taxa is measured from a faecal sample of the companion animal.

In certain embodiments, the bacterial taxa are measured at time points between about 1 week, about 2 weeks, about 2.1 days, about 2.8 days, about 1 month, about 56 days, about 2 months, about 3 months, about 4 months, about 84 days, about 5 months or about 6 months apart. In certain embodiments, the treatment regimen comprises a dietary regimen. In certain embodiments, the dietary regimen comprises administering an effective amount of any composition disclosed herein.

In certain embodiments, the amount of the bacterial taxa is determined using a DNA sequencing technique

In certain embodiments, the amount of the bacterial taxis determined using an RNA sequencing technique.

In certain embodiments, the amount of the bacterial taxa is determined using a microarray.

In certain embodiments, the amount of a bacterial taxa is determined using a polymerase chain reaction technique such as quantitative PCR.

## BRIEF DESCRIPTION OF DRAWINGS

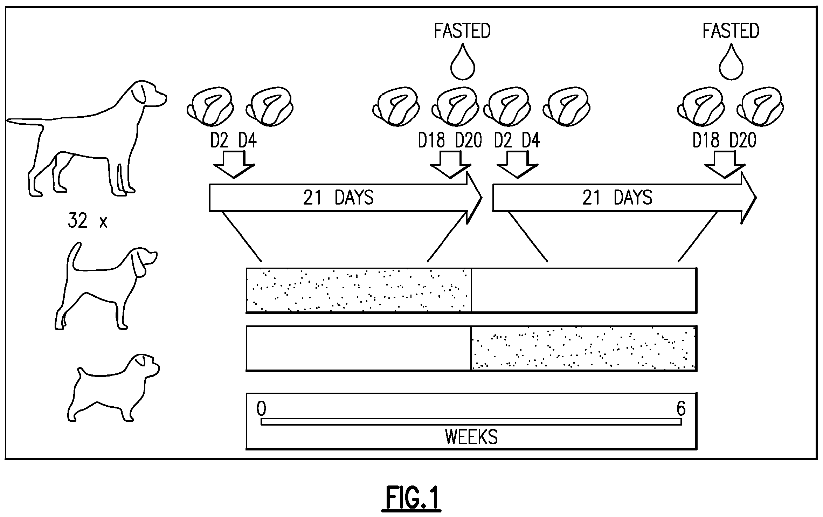

Figure 1: Figure 1 is a graphical depiction of the study design for comparison of the effects of individual dietary toppers on the faecal microbiome and a blood marker of intestinal permeability.

Figure 1: Figure 2 is a graphical depiction of the daily faecal wet weight and number of faeces measured as described in Example 1.

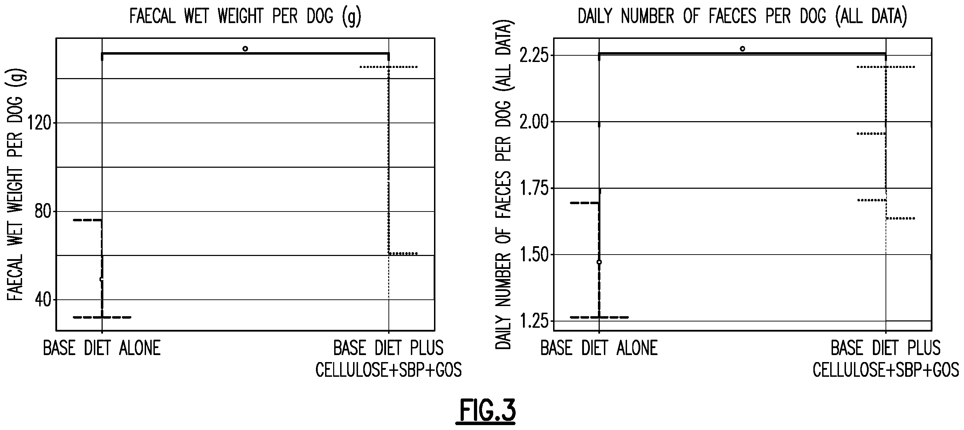

Figure 3: Figure 3 is a graphical depiction of the daily faecal wet weight and number of faeces measured as described in Example 1.

Figure 4: Figure 4 is a graphical depiction of the Shannon diversity of the samples collected from the cohort while the dogs received control diet and during the feeding of the microbiome supplement as described in Example 2.

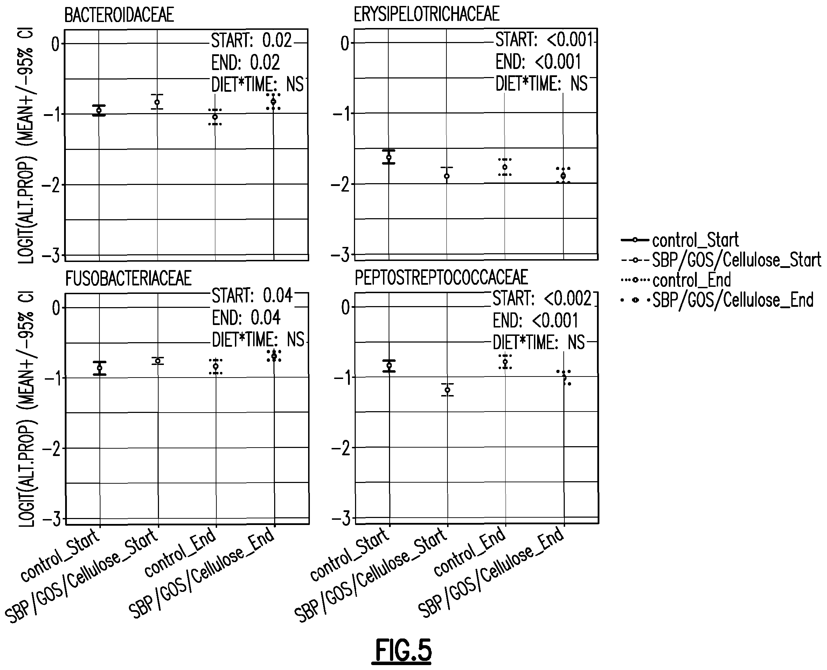

Figure 5: Figure 5 is a graphical depiction of the relative abundance (Logit-transformed) of bacterial families significantly affected by SBP/GOS/Cellulosc topper treatment (p-values indicated in the corner of each graph) as described in Example 2.

Figure 6: Figure 6 is a graphical depiction of the relative abundance (Logit-transformed) of bacterial genera significantly influenced by SBP/GOS/Cellulose topper treatment (p-values indicated in the corner of each graph) as described in Example 2.

## DISCLOSURE OF THE INVENTION

In some aspects, the present disclosure relates to compositions for use in altering the gut microbiota in companion animals. in order to impart a healthier status of the gut microbiome. The present disclosure also relates to methods for monitoring the influence of said compositions on host health status.

In some aspects, the present disclosure relates to compositions suitable for the administration of a companion animal. In some embodiments, the composition includes at least one of the ingredients selected from: galactooligosaccharides (GOS) of about 0.05 grams(g)/day to about 5.0 g/day; beet pulp and/or chicory pulp to a total amount of about 0.1 g/day to about 10 g/day; and cellulose from about 0.2 g/day to about 30.8 g/day. In one embodiment, the composition includes at least one of the ingredients is selected from: GOS of about 0.05g to about 3.6 g/day; beet pulp and/or chicory pulp to a total amount of about 1.65 g/day to about 9.52 g/day; and cellulose from about 5.32 g to about 25.98 g/day.

In other aspects, the present disclosure relates to methods for altering the gut microbiota and microbiomc of companion animals by a composition used as a dietary intervention such as a pet food product, a supplement, a pet food functional topper or booster, or a nutritionally complete dry kibble food for dogs containing a combination of the following GOS, cellulose, beet pulp and/or chicory pulp.

GOS is also known as oligogalactosyllactose, oligogalactosc. oligolactosc or transgalactooligosaccharides (TOS). They are produced through the enzymatic conversion of lactose and vary in chain length and type of linkage between the monomer units. They can additionally be found naturally in lower levels in plant and milk products or fractions. Cellulose is an insoluble substance which is the main constituent of plant cell walls and of vegetable fibres such as cotton. It is a polysaccharide consisting of chains of glucose monomers. Examples of celluloses suitable for this invention include but are not limited to vegetable fibres such as those found naturally in leaf. seed, straw, grass, and wood. Specifically, ingredients may include fibrous plant hydrocolloids that bind fat and water to create a bulking activity without anti-nutritional factors such as such as lettuce or leaf fibre or other plant fibres including pea fibre, husk fibres including psyllium husk, peanut husk and citrus peel fibre or pure natural grade fibres

In some embodiments, chicory pulp can be in the form of chicory pulp fibre. In some embodiments, beet pulp can be sugar beet pulp. and/or sugar beet pulp fibre.

Specific ingredients are described that enable a shift in the microbial community composition reflective of enhanced health and resilience of the gut microbiomc in companion animals. Thus, ingredient combinations for administration to companion animals through pet food products, treatments, supplements, boosters, or toppers are described along with methods of demonstrating, monitoring. and tracking the effect of the composition on the individual companion animal.

Experiments described below show that feeding the compositions described herein to a companion animal results in one or more of a decrease of unacceptable faeces, a decrease in faccal pH, an increase in the number of faeces, faeces wet weight that can also enhance and enable the removal of metabolic toxins, a decrease in lymphocyte counts and also the alteration of the gut microbiomc as demonstrated by the faecal microbiome in terms of the diversity of the feacal bacterial taxa, and specifically a decrease in bacterial diversity, an increase in the total bacterial taxa from families Bacteroidaceae and Fusobacteriaceae, a decrease in Erysipelotrichaceae, a decrease in Peptostreptococcaceae, as well as taxa from the *Peptostreptococcus* genus and an increase in the bacterial genus *Anaerobiospirillum*.

### *Monitoring*

In some aspects, the compositions described herein may be used as part of a monitoring regime for a companion animal. In other aspects, the present disclosure relates to a method of changing the microbiome of a companion animal by administering a composition as described herein to a companion animal. In one embodiment, there is a first step of determining the health of the companion animal's microbiome, and the composition is administered to the companion animal when there is a determination of a healthy or an unhealthy microbiome detected in the first step, preferably an unhealthy microbiome.

The methods of the present disclosure may also be performed more than once, for example two times, three times, four times, five times, six times, seven times or more than seven times. Tins allows the microbiome health status to be monitored overtime. This can be useful for example where a companion animal is receiving treatment to shift the microbiome or the owner finds it is desirable to improve the gastrointestinal health and resilience of the animal such that the occurrence of diarrhoea is reduced or even prevented or resolved where clinical signs are detected. The first time the method is performed, the microbiome health status is determined and, following the dietary intervention or administration of the composition of the invention, the method may be repeated to assess the influence of the composition on the microbiome. The microbiome health status may also be determined for the first time after the companion animal has received treatment and the method repeated afterwards to assess whether there is a change in the microbiome health status. In such case, a treatment effect would be indicated by the microbial taxa being altered wherein the levels of at one or more 2 of the bacterial taxa described herein are altered significantly compared to the previous measure or moving outside of normal ranges compared to an average range for a control dataset outside of the 95% range.

The method may be repeated several days, one week, two weeks, three weeks, one month, two months, three months, four months, five months, six months, 12 months, 18 months, 24 months, 30 months, 36 months, or more than 36 months apart.

### The companion animal

The composition of as disclosed herein can be used to change the microbiome of a companion animal. Companion animals would include pets and/or domestic animals such as canids and felines.

In some embodiments, the companion animal is a canid. This genus comprises domestic dogs (*Canis lupus familierris*), wolves, coyotes, foxes, jackals. dingoes and the invention can be used for all these animals. Most preferably, the subject is a domestic dog herein referred to simply as a dog.

The companion animal may be healthy. "Healthy" may refer to a companion animal who has not been diagnosed with a disease that is known to be related to the microbiome. Examples of such diseases include chronic and acute diarrhoea irritable bowel syndrome, ulcerative colitis, Crohn's disease and inflammatory bowel disease. Preferably, the companion animal does not suffer from dysbiosis.

There are numerous different breeds of domestic dogs which show a diverse liabitus. Different breeds also have different life expectancies with smaller dog breeds generally being expected to live longer than larger breeds. Accordingly, different breeds are considered to be junior, adult. senior or geriatric at different time points in their life. A summary of the different life stages is provided in the table below.

|        | Youth        | Adult       | Senior        | Geriatric   |
|--------|--------------|-------------|---------------|-------------|
| Toy    | Up to 7 years | 8-11 years  | 12-13 years   | 14+ years   |
| Small  | Up to 7 years | 8-11 years  | 12-13 years   | 14+ years   |
| Medium | Up to 5 years | 6-9 years   | 10-13 years   | 14+ years   |
| Large  | Up to 5 years | 6-9 years   | 10-11 years   | 12+ years   |

The distinction between toy, small, medium and large breeds is known in the art. In particular, toy breeds comprise distinct breeds such as Affenpinscher, Australian Silky Terrier, Bichon Frise, Bolognese, Cavalier King Charles Spaniel, Chihuahua, Chinese Crested, Coton De Tulear, English Toy Terrier, Griffon Bruxellois, Havanese, Italian Greyhound, Japanese Chin, King Charles Spaniel. Lowchcn (Little Lion Dog), Maltese, Miniature Pinscher. Papillon, Pekingese, Pomeranian, Pug, Russian Toy and Yorkshire Terrier.

Small breeds are larger on average than toy breeds with an average body weight of up to about 10kg. Exemplary breeds include French Bulldog, Beagle. Dachshund, Pembroke Welsh Corgi, Miniature Schnauzer, Cavalier King Charles Spaniel, Shih Tzu, and Boston Terrier.

Medium dog breeds have an average weight of about 10 to about 26kg, preferably 10 to 25 kg. These dog breeds include Bulldog, Cocker Spaniel, Shetland Sheepdog, Border Collie. Basset Hound. Siberian Husky and Dalmatian.

Large breeds arc those with an average body weight of at or greater than about 25kg, preferably at least 27 kg. Examples include Great Dane, Ncapolitan mastiff. Scottish Deerhound, Dogue de Bordeaux, Newfoundland, English mastiff, Saint Bernard, Leonberger and Irish Wolfhound.

Cross-breeds can generally be categorised into toy, small, medium, and large dogs depending on their body weight.

### The sample

The methods of the present disclosure generally use a fecal sample or a sample from the gastrointestinal lumen of the companion animal. Fecal samples are convenient because their collection is non-invasive, and it also allows for easy repeated sampling of individuals over a period of time. The invention can also be used with other samples, such as ileal, jejunal, duodenal samples and colonic samples.

The sample may be a fresh sample. The sample may also be frozen or stabilised by other means such as addition to preservation buffers or by dehydration using methods such as freeze drying before use in the methods of the invention.

Before use in the methods of the present disclosure, the sample will generally be processed to extract DNA. Methods for isolating DNA are well known in the art, as reviewed in reference (Hart *et al.* (2015) PLoS One. Nov 24;10(11):e0143334), for example. Suitable methods include, for instance, the QIAamp Power Faecal DNA kit (Qiagen). To date, there remains a need for novel methods and compositions for treating intestinal dysbiosis and other intestinal disorders that target gut microbiome for improvement of health and gastrointestinal resilience.

For clarity and not by way of limitation, the detailed description of the presently disclosed subject matter is divided into the following subsections:

1. Microbiome health and good faecal quality,
2. Compositions;
3. Treatment methods; and
4. Testing, tracking and monitoring methods

## 1. Microbiome health and good faecal quality

In certain embodiments, the change in a bacterial taxa of a companion animal can be used to indicate its intestinal health and likely faecal quality. In certain embodiments, the bacterial taxa is associated to a healthy status or an intestinal dysbiosis in a subject, or good or bad faecal quality.

In certain embodiments, a change or stabilisation or reduced variability in the diversity of the faecal microbial community or microbiota Shannon diversity levels after being fed a composition of the present invention could indicate an improvement in the health status of a companion animal. In certain embodiments, a change in Bacteroidaceae, Eryslpeiotrichaceae, Fusobacteriaceae and Peptostreptococcaceae levels after being fed a composition of the present disclosure indicate an improvement in the health status of a companion animal. In one embodiment, the Bactcroidacea and Fusobacercicaea levels increase significantly after feeding a composition of the present disclosure. In another embodiment, the Erysipelotrichaeceae and Peptostreptococcaecea levels decrease significantly after feeding a composition of the present disclosure.

In certain embodiments, a change in the genus *Peptostreptococcus* species or in the taxa from the *Escherichia / Shigella* group or in *Anaerobiospirillium* sp. after being fed a composition of the present invention could indicate an improvement in the health status of a companion animal.

## 2. Compositions

In some aspects, the present disclosure relates to compositions suitable for administration to a companion animal, said compositions including at least one of the ingredients selected from: galactooligosaccharides of about 0.05 g/day to about 5.0 g/day; beet pulp and/or chicory pulp to a total amount of about 0.1 g/day to about 10 g/day; and cellulose from about 0.2 g/day to about 30.8 g/day.

In one embodiment, the composition is a food, nutraceutical, pharmaceutical, supplement, booster or topper. In one embodiment, such compositions are suitable for administration to a companion animal.

"Companion animal", as used herein, includes any animal that can be found in a domestic setting, including mammals such as canids and felines.

In one embodiment, the compositions are suitable for use in a medicament.

In one embodiment, the compositions are suitable for use in treating gastro-intestinal dysbiosis in a companion animal. In one embodiment, the composition is suitable for use in altering the microbiome in a companion animal. In one embodiment, the composition is suitable for use to increase the numbers of Bacteroidaceae, Fusobacteriaceaeand Anaerobiospirillumpresent in the gastro-intestinal tract or faeces of a companion animal compared to the number of said bacteria present in the companion animal before administration of the composition. In one embodiment, the composition is suitable for use to decrease the number of Erysipelotrichaeceae and Peptostreptococcaceae. In one embodiment, the composition is for use to decrease the fecal pH of the companion animal.

In certain embodiments, the composition comprises an effective amount of pulp. In certain embodiments, the pulp is beet pulp such as sugar beet pulp, preferably raw sugar beet pulp. In another embodiment, the pulp may be chicory pulp. preferably chicory pulp fibre. In certain embodiments, the pulp is cooked or sterilized or included with an extruded or a processed product. In one embodiment, the pulp of the composition may originate from more than one plant e.g., chicory and beet.

In certain embodiments, the pulp is at a concentration between about 0.5% w/w and about 10% w/w, between about 0.5% w/w and about 5% w/w, between about 0.5% w/w and about 4% w/w, between about 0.5% w/w and about 3% w/w, between about 0.5% w/w and about 2% w/w. between about 0.5% w/w and about 1.5% w/w, between about 0.5% w/w and about 1.2% w/w, between about 0.5% w/w and about 1 % w/w, between about 0.5% w/w and about 0.9% w/w, or between about 0.5% w/w and about 0.8% w/w. In certain embodiments, the pulp is at a concentration between about 0.8% w/w and about 10% w/w, between about 0.8% w/w and about 5% w/w, between about 0.8% w/w and about 4% w/w, between about 0.8% w/w and about 3% w/w, between about 0.8°% w/w and about 2% w/w, between about 0.8% w/w and about 1.5% w/w, between about 0.8%, w/w and about 1% w/w, between about 1% w/w and about 10% w/w, between about 1% w/w and about 5% w/w, between about 2% w/w and about 5% w/w, or between about 1% w/w and about 2% w/w. In certain embodiments, the pulp is at a concentration of about 0.8% w/w.

In certain embodiments, the composition comprises an effective amount of any bacterium disclosed herein that is associated to heathy intestine status in a subject.

In certain embodiments, the bacterium comprised in the composition is between about 1 thousand CFU and about 10 trillion CPU. In certain embodiments, the bacterium is between about 1 thousand CFU and about 1 trillion CFU, between about 1 million CFU and about 1 trillion CFU, between about 100 million CFU and about 100 billion CPU. between about 1 billion CPU and about 1 trillion CPU, between about 1 billion CFU and about 100 billion CFU. between about 100 million CFU and about 100 billion CFU, between about 1 billion CFU and about 50 billion CFU, between about 100 million CFU and about 50 billion CPU. or between about 1 billion CFU and about 10 billion CFU. In certain embodiments, the bacterium comprised in the composition is at least about 1 thousand CFU. at least about 1 million CFU, at least about 10 million CFU, at least about 100 million CFU, at least about 1 billion CFU, at least about 10 billion CFU, at least about 100 billion CFU or more.

In certain embodiments, the composition further comprises an effective amount of pulp such as chicory pulp or beet pulp (e.g., sugar beet pulp).

In certain embodiments, the composition is a dietary supplement, for example, applied on top of the composition, as a pet food topper or subsequently mixed throughout the product. In certain embodiments, the composition is a treat product or a chew or a kibble-based treat or complementary product. In certain embodiments, the composition is a cat food product or a dog food product. In certain embodiments, the food product is a dog food product. In certain embodiments, the composition is a dry pet food product. In certain embodiments, the composition is a wet pet food product.

In certain embodiments, a formulation of the presently disclosed subject matter can further comprise an additional active agent. Non-limiting examples of additional active agents that can be present within a formulation of the presently disclosed subject matter include a nutritional agent (e.g., amino acids, peptides, proteins, fatty acids, carbohydrates, sugars, nucleic acids, nucleotides, vitamins, minerals, *etc.*), a prebiotic, a probiotic. an antioxidant, and/or an agent that enhances the microbiome, improves gastrointestinal health and improves animal health.

In certain embodiments, the composition comprises one or more probiotic. In certain embodiments, the probiotic is an animal probiotic. In certain embodiments, the animal probiotic is a feline probiotic. In certain embodiments, the animal probiotic is a canine probiotic. In certain embodiments, the probiotic is from the family Bacteroidaceae and/or Fusobacteriaceae or the genus Anacrobiosprillum. In certain embodiments, the animal priobitic is further selected from *Bifidobacterium, Lactobacillus, lactic acid bacterium* and/or *Enterococcus.* In certain embodiments, the probiotic is selected from any organism from lactic acid bacteria and more specifically from the following bacterial genera; *Lactococcus spp., Pediococcus spp., Bifidobacterium spp.* (e.g., *B. longum B. bifidum, B. pseudolongum, B. animalis, B infantis). Lactobacillus spp. (e.g., L. bulgaricus, L. acidophilus, L. brevis, Z casei, L. rhamnosus, L. plantarum, L. reuteri, Z. fermentum, Enterococcus spp.* (e.g., *E. faecium), Prevotella spp., Fusobacterium spp. Alloprevotella spp.* and any combination thereof. In certain embodiments, the probiotic is administered to a companion animal in an amount of from about 1 colony forming unit (CFU) to about 100 billion CFUs per day for the maintenance of the GI microflora or the microbiome or gastrointestinal health. In certain embodiments, the probiotic is administered to a companion animal in an amount of from about 1 colony forming unit (CFU) to about 20 billion CPUs per day for the maintenance the GI microflora or the microbiome or gastrointestinal health. In certain embodiments, the probiotic is administered to a companion animal in an amount of from about 1 billion CFUs to about 20 billion CPUs per day for the maintenance of GI microflora. In certain embodiments, the probiotic is administered to a companion animal in amounts of from about 0.01 billion to about 100 billion live bacteria per day. In certain embodiments, the probiotic is administered to a companion animal in amounts of from about 0.1 billion to about 10 billion live bacteria per day. In certain embodiments, the probiotic is administered to a companion animal in amounts of from about 1x104 CFU to $1 \times 10^{14}$ CFU per day. In certain embodiments, an additional prebiotic can be included, such as fructooligosaccharides (FOS). xylooligosaccharides (XOS), soybean oligosaccharides (SO), glucans. galactans. arabinogalactan, inulin and/or mannooligosaccharides. In certain embodiments, the additional prebiotic is administered in amounts sufficient to positively stimulate the microbiome or the GI microflora and/or cause one or more probiotic to proliferate.

In certain embodiments, the composition can further contain additives known in the art. In certain embodiments, such additives are present in amounts that do not impair the purpose and effect provided by the presently disclosed subject matter. Examples of contemplated additives include, but are not limited to, substances that arc functionally beneficial to improving health, substances with a stabilizing effect, organoleptic substances, processing aids, substances that enhance palatability, coloring substances, and substances that provide nutritional benefits. In certain embodiments, the stabilizing substances include, but are not limited to, substances that tend to increase the shelf life of the product. In certain embodiments, such substances include, but are not limited to, preservatives, synergists and sequestrants, packaging gases, stabilizers, emulsifiers, thickeners, gelling agents, and humectants. In certain embodiments, the emulsifiers and/or thickening agents include, for example, gelatin, cellulose ethers, starch, starch esters, starch ethers, and modified starches.

In certain embodiments, the additives for coloring, palatability, and nutritional purposes include, for example, colorants; iron oxide, sodium chloride, potassium citrate, potassium chloride, and other edible salts; vitamins; minerals: and flavoring. The amount of such additives in a product typically is up to about 5% (dry basis of the product).

In certain embodiments, the composition is a dietary supplement. In certain embodiments, the dietary supplements include, for example, a feed used with another feed to improve the nutritive balance or performance of the total. In certain embodiments, the supplements include compositions that are fed undiluted as a supplement to other feeds, offered free choice with other parts of an animal's ration that are separately available, or diluted and mixed with an animal's regular feed to produce a complete feed. The AAFCO, for example, provides a discussion relating to supplements in the American Feed Control Officials, Incorp. Official Publication, p. 220 (2003). Supplements can be in various forms including, for example, powders, liquids, syrups, pills, tablets, encapsulated compositions. *etc.*

In certain embodiments, the composition is a treat. In certain embodiments, treats include, for example, compositions that are given to an animal to entice the animal to eat during a non-mealtime. In certain embodiments, the composition is a treat for canines include, for example, dog bones. Treats can be nutritional, wherein the product comprises one or more nutrients, and can, for example, have a composition as described above for food. Non-nutritional treats encompass any other treats that are non-toxic.

In certain embodiments, a bacterium and/or sugar beet pulp of the presently disclosed subject matter can be incorporated into the composition during the processing of the formulation, such as during and/or after mixing of other components of the product Distribution of these components into the product can be accomplished by conventional means.

In certain embodiments, composition of the presently disclosed subject matter can be prepared in a canned or wet form using conventional companion animal food processes In certain embodiments, ground animal (e.g., mammal, poultry, and/or fish) proteinaceous tissues are mixed with the other ingredients, such as milk fish oils, cereal grains, other nutritionally balancing ingredients, special purpose additives (e.g., vitamin and mineral mixtures, inorganic salts, cellulose and beet pulp. bulking agents, and the like); and water that sufficient for processing is also added. These ingredients are mixed in a vessel suitable for heating while blending the components. Heating of the mixture can be affected using any suitable manner, such as, for example, by direct steam injection or by using a vessel fitted with a heat exchanger. Following the addition of the last ingredient, the mixture is heated to a temperature range of from about 50° F to about 212° F. Temperatures outside this range are acceptable but can be commercially impractical without use of other processing aids. When heated to the appropriate temperature, the material will typically be in the form of a thick liquid. The thick liquid is filled into cans. A lid is applied, and the container is hermetically sealed. The scaled can is then placed into conventional equipment designed to sterilize the contents. This is usually accomplished by heating to temperatures of greater than about 230° F for an appropriate time, which is dependent on, for example, the temperature used and the composition.

In certain embodiments, the composition of the presently disclosed subject matter can be prepared in a dry form using conventional processes. In certain embodiments, dry ingredients, including, for example, animal protein sources, plant protein sources, grains, *etc.,* are ground and mixed together. In certain embodiments, moist or liquid ingredients, including fats, oils, animal protein sources, water, *etc.,* are then added to and mixed with the dry mix. In certain embodiments, the mixture is then processed into kibbles or similar dry pieces. In certain embodiments, composition is a kibble. In certain embodiments, kibble is formed using an extrusion process in which the mixture of dry and wet ingredients is subjected to mechanical work at a high pressure and temperature and forced through small openings and cut off into kibble by a rotating knife. In certain embodiments, the wet kibble is then dried and optionally coated with one or more topical coatings which can include, for example, flavors, fats, oils, powders, and the like. In certain embodiments, kibble can also be made from the dough using a baking process, rather than extrusion, wherein the dough is placed into a mold before dry-heat processing

In certain embodiments, treats of the presently disclosed subject matter can be prepared by, for example, an extrusion or baking process similar to those described above for dry food.

In certain embodiments, the composition of the present invention further comprises Royal Canin™ Indoor Life pet food.

### 3. Treatment Methods

In certain non-limiting embodiments, the presently disclosed subject matter provides methods for enhancing or improving the microbiome, for improving intestinal health and/or treating an intestinal dysbiosis of a subject in need thereof. In certain embodiments, the subject is a companion animal, e.g., a dog or a cat. In certain embodiments, the method can improve immunity, digestive function, fecal quality and amount. and/or reduce dysbiosis of a companion animal.

In certain embodiments, the method comprises administering to the subject an effective amount of any presently disclosed compositions. In certain embodiments, the method further comprises monitoring any presently disclosed bacterial taxa in the subject. In certain embodiments, the bacterial taxa are measured in a fecal sample of the subject. In certain embodiments, the bacterial taxa are measured in a sample from the intestines of the subject.

In certain embodiments, the composition can be administered to a subject from 20 times per day to once per day, from 10 times per day to once per day, or from 5 times per day to once per day. In certain embodiments, the composition can be administered to a subject once per day, twice per day, thrice per day, 4 times per day, 5 times per day, 6 times per day, 7 times per day, 8 times per day, 9 times per day, 10 or more times per day. In certain embodiments, the composition can be administered to a subject once per two days, once per three days, once per four days, once per five days, once per six days, once a week, once per two weeks, once per three weeks, or once per month. In certain embodiments, the composition can be administered to an animal in a constant manner, e.g.. where the animal grazes on a constantly available supply of the subject composition.

In certain embodiments, the dosage of the composition is between about 1 mg/kg body weight per day and about 5000 mg/kg body weight per day. In certain embodiments, the dosage of the pet food product is between about 5 mg/kg body weight per day and about 1000 mg/kg body weight per day, between about 10 mg/kg body weight per day and about 500 mg/kg body weight per day, between about 10 mg/kg body weight per day and about 250 mg/kg body weight per day, between about 10 mg/kg body weight per day and about 200 mg/kg body weight per day. between about 20 mg/kg body weight per day and about 100 mg/kg body weight per day, between about 20 mg/kg body weight per day and about 50 mg/kg body weight per day or any intermediate range thereof. In certain embodiments, the dosage of the pet food product is at least about 1 mg/kg body weight per day, at least about 5 mg/kg body weight per day, at least about 10 mg/kg body weight per day, at least about 20 mg/kg body weight per day, at least about 50 mg/kg body weight per day, at least about 100 mg/kg body weight per day, at least about 200 mg/kg body weight per day or more. In certain embodiments, the dosage of the pet food product is no more than about 5 mg/kg body weight per day. no more than about 10 mg/kg body weight per day, no more than about 20 mg/kg body weight per day, no more than about 50 mg/kg body weight per day, no more than about 100 mg/kg body weight per day, no more than about 200 mg/kg body weight per day, no more than about 500 mg/kg body weight per day or more.

In certain embodiments, the amount of composition decreases over the course of feeding a companion animal. In certain embodiments, the concentration of the composition increases over the course of feeding a companion animal. In certain embodiments, the concentration of the composition is modified based on the age of the companion animal.

In certain non-limiting embodiments, the presently disclosed subject matter provides for a method for determining an intestinal health status in a companion animal in need thereof.

In certain embodiments, the amount of a bacterial taxa can be determined by any method known in the art. In certain embodiments, the method includes, but is not limited to, antibody-based detection methods detecting a protein/antigen associated with the microorganism, e.g., an enzyme-linked immunosorbent assay (ELISA), flow cytometry, western blot; and methods for detecting a 16s rRNA associated with the microorganism, e.g., real-time polymerase chain reaction (RT-PCR), quantitative polymerase chain reaction (qPCR), DNA sequencing and microarray analyses. In certain embodiments, the microarray comprises probes for detecting any of the intestinal microorganism disclosed herein.

In certain embodiments, the treatment regimen can be any treatment regimen of dysbiosis known in the art. In certain embodiments, the treatment regimen comprises a treatment method disclosed herein.

In certain embodiments, the amount of the intestinal bacterium is measured from a fecal sample of the subject.

### 4. Testing, Tracking and Monitoring Methods

In addition, or alternatively, the companion animal's microbiome health may be assessed by determining the diversity of bacterial species within a companion animal's microbiome. To this end, the diversity index of the bacterial species within the companion animal's microbiome is determined and compared to the diversity index of a control data set. Over repeated samplings the stability of the microbiome is assessed where stability in the diversity is a sign of the health of the microbiome.

*The control data set*

The abundance of the bacterial species is compared to a control data set from a companion animal, e.g., a canid, with a similar chronological age, e.g., a puppy, an adult canid, a senior canid or a geriatric canid. PCT/US2020/014292 or WO2020/150712 which are hereby incorporated in their entirety by reference provide suitable control data sets against which the microbiomc composition from the canid may be compared.

Alternatively, or in addition, a control data set may be prepared. To this end, the microbiome of two or more (e.g., 3. 4, 5, 10, 15, 20 or more) healthy canids may be analysed for the abundance of the species contained in the microbiome. A healthy canid in this context is a canid who has not been diagnosed with a disease that is known to affect the microbiome. Examples of such diseases include irritable bowel syndrome, ulcerative colitis, Crohn's and inflammatory bowel disease. Preferably, the canid does not suffer from dysbiosis. Dysbiosis refers to a microbiome imbalance inside the body, resulting from an insufficient level of keystone bacteria (e.g., *bifidobacteria,* such as *B. longiini subsp. infantis*) or an overabundance of harmful bacteria in the gut. Methods for detecting dysbiosis are well known in the art. The two or more canids will generally be from a particular life stage. For example, they may be puppies, adult canids. senior canids or geriatric canids. This is useful because the microbiome changes in a canid's lifetime and the microbiome therefore needs to be compared to a canid at the same life stage. Where the canid is a dog, the control data set may further be from a dog of the same breed or, where the dog is a mongrel, the same breed as one of the direct ancestors (parents or grandparents) of the dog.

Specific steps to prepare the control data set may comprise analysing the microbiome composition of at least two (e.g., 3, 4, 5, 6, 7. 8 9, 10. 15, 20 or more) puppies, and/or at least two (e.g., 3, 4, 5, 6. 7, 8 9. 10, 15. 10 or more) adult canids, and/or at least two (e.g., 3, 4, 5. 6, 7, 8 9. 10, 15. 20 or more) senior canids and/or at least two (e.g., 3, 4, 5, 6. 7, 8 9, 10. 15, 20 or more) geriatric canids; determining the abundance of bacterial species (in particular those discussed above); and compiling these data into a control data set.

For embodiments where the diversity index of the microbiome is determined, the control data set may be prepared in a similar manner. In particular, the diversity index can be determined in two or more healthy canids at a particular life stage (puppy, adult, senior or geriatric). The results can then be used to identify the mean range for the diversity index in a canid at that life stage.

It will be understood that the control data set does not need to be prepared every time the method of the invention is performed. Instead, a skilled person can rely on an established control set.

The control data set may also comprise data from the same companion animal at different time points.

Techniques which allow a skilled person to detect and quantitate bacterial taxa are well known in the art. These include, for example, 16S rDNA amplicon sequencing, shotgun sequencing, metagenome sequencing, Illumina sequencing, and nanopore sequencing. Preferably, the bacterial taxa are determined by sequencing the 16s rDNA sequence.

In some embodiments, the bacterial taxa are determined by sequencing the V4-V6 region, for example using Illumina sequencing.

The bacterial species may also be detected by other means known in the art such as, for example, RNA sequencing, protein sequence homology or other biological marker indicative of the bacterial species.

The sequencing data can then be used to determine the presence or absence of different bacterial taxa in the sample. For example, the sequences can be clustered at 98%. 99% or 100% identity and abundant taxa (e.g.. those representing more than 0.001 of the total sequences) can then be assessed for their relative proportions. Suitable techniques are known in the art and include, for example, logistic regression, partial least squares discriminate analysis (PLS-DA) or random forest analysis and other multivariate method.

*General*

The term "comprising" encompasses "including" as well as "consisting" e.g., a composition "comprising" X may consist exclusively of X or may include something additional e.g., Y + Y.

The term "about" in relation to a numerical value x is optional and means, for example, $x \pm 10\%$.

The word "substantially" does not exclude "completely" e.g., a composition which is "substantially free" from Y may be completely free from Y. Where necessary, the word "substantially" may be omitted from the definition of the disclosed methods and compositions.

References to a percentage sequence identity between two nucleotide sequences means that, when aligned, that percentage of nucleotides are the same in comparing the two sequences. This alignment and the percent homology or sequence identity can be determined using software programs known in the art. A preferred alignment is determined using the BLAST (basic local alignment search tool) algorithm or the Smith-Waterman homology search algorithm using an affine gap search with a gap open penalty of 12 and a gap extension penalty of 2, BLOSUM matrix of 62. The alignment may be over the entire reference sequence, i.e., it may be over 100% length of the sequences disclosed herein.

Unless specifically stated, a process or method comprising numerous steps may comprise additional steps at the beginning or end of the method or may comprise additional intervening steps. Also, steps may be combined, omitted or performed in an alternative order, if appropriate.

Various embodiments of the disclosed compositions and methods arc described herein. It will be appreciated that the features specified in each embodiment may be combined with other specified features, to provide further embodiments. In particular, embodiments highlighted herein as being suitable, typical, or preferred may be combined with each other (except when they are mutually exclusive).

## MODES FOR CARRYING OUT THE IN VENTION

### *Example 1: Fibre based composition for influencing faecal pH through the gut microbiota, and gastrointestinal health and resilience*

### Introduction

Colonic microbial communities are vital to the integrity of the gut barrier as well as providing essential metabolites to the host. Whilst the microbiota exists with altered bacterial proportions represented along the intestinal tract. studies of the gut microbiome in pets are predominantly conducted using a faecal surrogate sample. This represents a similar approach to the non-invasive human gut microbiome approaches where faecal samples are analysed as a standard proxy for luminal samples in order to gain insights on the microbiome of the gut.

A feeding study was conducted to identify whether a prebiotic/soluble and insoluble fibre combination in a cohort of animals. The composition was formulated from feed grade cellulose, sugar beet pulp and galactooligosaccharides (GOS) and the study aimed assess whether an altered gut microbial composition or microbiome was associated with feeding the ingredients in an unprocessed dietary topper format and additionally whether effects on systemic health were observed. The systemic/physiological measures included faecal consistency and defaecation characteristics complete blood cell counts (CBC) and blood endotoxin (lipopolysaccharide) levels.

### Methods

A cohort of 32 dogs were assigned to the study which utilised a crossover design comparing test and control treatments to assess whether the ingredients fed as a dietary topper alter the composition or microbiota signatures in dogs compared to a control reference diet. In addition to the assessment of faecal microbial populations whether systemic treatment effects were observed was also assessed through haematological measurement of blood leaukocyte populations that may be indicative of immune effects induced through feeding of the composition.

A consistent premium commercial dry pet food diet (RC Indoor Adult Dog) was fed throughout the study. At the start of each study phase the control diet or the control diet with one of the test pet food dietary topper composition (cellulose, sugar beet pulp and GOS) applied to the diet was fed to the dogs daily at each meal. The animals were fed twice per day and feeding of each treatment (control base diet or base diet with the fibre dietary topper: table 2.1) was conducted for a period of 21 days to allow for an extended period of faeces quality data collection and for stabilisation of the gastrointestinal microbiota following the dietary change.

Faeces quality was recorded daily and faeces samples for assessment of the microbiome were collected on days 2-4 and 18-20 (or +1 day if samples are not produced for collection on target days). Fresh faecal samples were collected from each dog no more than 15 minutes after defecation and will be stored at -80 degrees centigrade prior to processing for analysis of the microbial composition in faeces using deep sequencing approaches to determine the bacterial community composition through amplicon sequencing of the 16S rDNA region V3-6.

A 6 ml fasted blood sample was collected once at the end of each phase from a consistent subset of the cohort (10 animals) To assess complete blood cell counts (CBC) and blood endotoxin (lipopolysaccharide) levels.

Table 2 7 Ingredient composition cellulose, sugar beet pulp and GOS

| Dog size and topper category | Bodyweight (kg) | Range per day Sugar beet pulp (g) | Range per day Sugar beet pulp (g) | Range per day Cellulose (g) | Range per day Cellulose (g) | Range per day GOS | Range per day GOS |
|---|---|---|---|---|---|---|---|
| | | Min | Max | Min | Max | Min | Max |
| Small dog | 5-10kg | 165 | 5.38 | 5 32 | 12.68 | 0.05 | 3.6 |
| Medium dog | 10-25kg | 2.14 | 6.36 | 6.89 | 15.86 | 0.1 | 3 52 |

(continued)

| Dog size and topper category | Bodyweight (kg) | Range per day Sugar beet pulp (g) | Range per day Sugar beet pulp (g) | Range per day Cellulose (g) | Range per day Cellulose (g) | Range per day GOS | Range per day GOS |
|---|---|---|---|---|---|---|---|
| | | Min | Max | Min | Max | Min | Max |
| Large dog | 25kg + | 3.72 | 9.52 | 11.95 | 25.98 | 1 44 | 3.52 |
| | | | | | | | |
| Optimal Ratio | - | 1 | | 3.3 | | 03 | |

## Statistical methods for the experimental approach

The trial followed a two-way crossover design with 21 days phases. Thirty-two senior dogs were split into two experimental groups; A and B. Group A received the test diet (Base + cellulose, sugar beet pulp and GOS) during the first phase followed by the control diet (base only) and group B received the control diet during the first phase followed by the test diet (Base + cellulose, sugar beet pulp and GOS).

Statistical models: For Daily Intake (g) and Faeces pH, linear mixed effects models were fit modelling the response against diet, time in phase and their interaction as fixed effects and animal as the random effect. For determining significance of the Proportion Unacceptable Faeces (% diarrhoeal faeces; all data) and Proportion Unacceptable Faeces (last 14 days of each phase), binomial generalised linear mixed effects models were fit modelling the binary response (acceptable = 0. unacceptable = 1) against diet as the fixed effect and pen pair with phase day nested in pen pair as the random structure. For all other measures, linear mixed effects models were fit modelling the response against diet as the fixed effect with animal as the random effect for blood measures and pen pair with phase day nested in pen pair as the random structure for faecal wet weight measures. Visual inspection of (linear mixed) model residuals was used to assess conformity with model assumptions. Where required a $\log_{10}$ transformation was applied to meet the assumption of normality.

Outputs: From all models, means with 95% confidence intervals are reported for each diet or for each time within each diet if appropriate. Where $\log_{10}$ transformations were applied, the reported means and CIs were back transformed to the original scale. For the binomial GLMMs, reported means and CIs represent proportions. Contrasts were applied between diets or. if appropriate, between diets within each time. between times within each diet and between times comparisons compared between diets. These contrasts are reported as fold changes with 95% confidence intervals and $p$-values for a difference from 1 where a $\log_{10}$ transformation was applied, as odds ratios with 95% confidence intervals and $p$-values for a difference from 1 for the binomial GLMMs and as differences with 95% confidence intervals and $p$-values for a difference from 0 for all other models.

## Results

A total of 32 dogs were assigned to and completed the study. The tables below show the balance of breed, sex, age and location between the two experimental groups. All animals enrolled in the study had been neutered. The dogs were all pure bred and were of three breeds, namely Beagle. Brittany Spaniel and Labrador Retriever The animals assigned to the study were between 8.9 and 12.9 years of age and 14 were female while 18 were male. The groups are well balanced for sex and breed size.

For each measure, the means and contrasts are reported in the tables along with a figure showing the means and confidence intervals. Significant contrasts are highlighted on the figure with stars and bars.

Table 2.2. Cohort details: gender

| Gender | | |
|---|---|---|
| **Group** | **Female** | **Male** |
| A | 7 | 9 |
| B | 7 | 9 |

Cohort details:

| Dog breed | | | |
|---|---|---|---|
| Group | Beagle | Brittany | Labrador Retriever |
| A | 8 | 3 | 5 |
| B | 6 | 5 | 5 |

Cohort details:

| Dog age (years) | | | | |
|---|---|---|---|---|
| Group | Minimum Age | Mean Age | Median Age | Maximum Age |
| A | 10.3 | 11.4 | 11.5 | 12.9 |
| B | 8.9 | 10.5 | 10.7 | 12.0 |

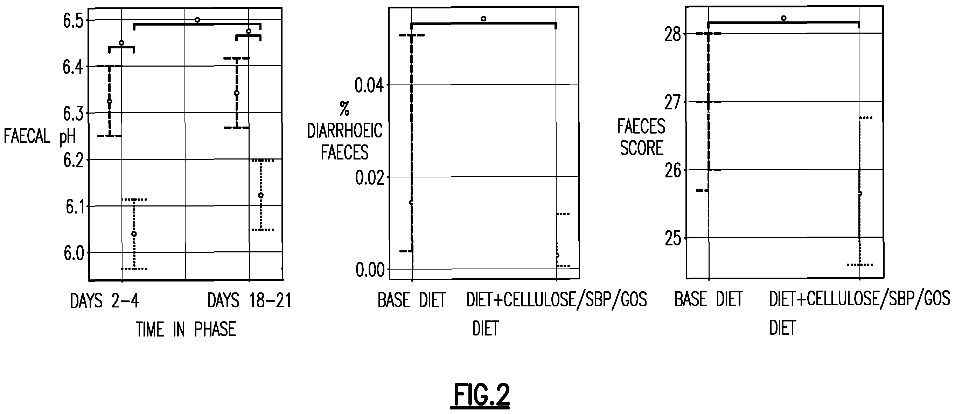

Faecal scores and % of unacceptable faeces were significantly decreased in Topper-fed dogs (p<0.001; Figure 2). Faecal pH was significantly reduced in Topper-fed dog at the start and the end of phase (p<0.001).

Faeces quality, the percentage of unacceptable faeces and Faecal pH were significantly reduced by the cellulose, sugar beet pulp and GOS composition indicating a change in the microbial metabolites and the microbiome of the dogs. Daily faecal wet weight and number of faeces were also significantly increased in the dogs when they received the topper cellulose, sugar beet pulp and GOS composition (p<0.001; Figure 2) while Daily feed intake was the same between treatment group.

Faecal pH

| Diet | Time in Phase | Mean (95% CI) |
|---|---|---|
| Royal Canin Indoor Life | baseline | 6.32 (6.25, 6.4) |
| Royal Canin Indoor Life | endpoint | 6.34 (6.27, 6.42) |
| Royal Canin Indoor Life plus SBP/GOS/Cell | baseline | 6.04 (5.97, 6.11) |
| Royal Canin Indoor Life plus SBP/GOS/Cell | endpoint | 6.12 (6.05, 6.2) |

Contrasts in Faecal pH

| Diet | Time in Phase | Mean (95% CI; *<0.05, **<0.001) |
|---|---|---|
| Royal Canin Indoor Life plus SBP/GOS/Cell-Royal Canin Indoor Life | Start of phase | -0.284 (-0.359, -0.21)** |
| Royal Canin Indoor Life plus SBP/GOS/Cell-Royal Canin Indoor Life | End of Phase | -0.221 (-0.296. -0.146) ** |
| Royal Canin Indoor Life | endpoint-baseline | 0.0183 (-0.0564, 0.093) |
| Royal Canin Indoor Life plus SBP/GOS/Cell | endpoint-baseline | 0.0817 (0.00696, 0.156)* |

(continued)

| Diet | Time in Phase | Mean (95% CI; *<0.05, **<0.001) |
|---|---|---|
| Royal Canin Indoor Life plus SBP/GOS/Cell-Royal Canin Indoor Life | endpoint-baseline | 0.0633 (-0.0423, 0.169) |

Table 2.3: Complete blood counts from 10 dogs sampled at the end of each phase * p<0.05

| | | Topper | | | |
|---|---|---|---|---|---|
| Measure | Control | (SBP+GOS) | Estimate (3 s.f.) | 95% CI | p-value |
| WBC | 7.96 | 7.81 | 0.98 | (0.915, 1.05) | 0.573 |
| % Neutrophils | 60.7 | 61.0 | 1.01 | (0.986, 1.(2) | 0.589 |
| % Lymphocytes | 26.1 | 25.1 | -1.06 | (-2.79, 0.672) | 0.230 |
| % Monocytes | 7.77 | 8.40 | 1.08 | (0.953, 1.23) | 0.229 |
| % Eosinophils | 4.93 | 5.00 | 0.07 | (-0.42, 0.56) | 0.779 |
| % Basophils | 0.14 | 0.17 | 0.03 | (-0.0419, 0.102) | 0.413 |
| Neutrophils | 4.83 | 4.76 | 0.986 | (0.908, 1.07) | 0.727 |
| Lymphocytes | 2.10 | 1.97 | -0.124 | (-0.244, -0.00419) | 0.042* |
| Monocytes | 0.64 | 0.69 | 0.0476 | (-0.0605, 0.156) | 0.388 |
| Eosinophils | 0.47 | 0.37 | 0.788 | (0.508. 1.22) | 0.289 |
| Cholesterol | 261.2 | 256.0 | 0.98 | (0.939, 1.02) | 0.356 |

Blood lymphocyte counts were significantly reduced in dogs when they received the dietary topper of cellulose, sugar beet pulp and GOS at the end of phase (p=0.042). No significant effect of treatment on other blood parameters measured.

Conclusions

Faeces quality, the percentage of unacceptable faeces and faecal pH were significantly reduced by the cellulose, sugar beet pulp and GOS composition indicating a change in the microbial metabolites and the microbiome of the dogs. Daily faecal wet weight and number of faeces were also significantly increased in the dogs when they received the topper cellulose, sugar beet pulp and GOS composition. Topper and booster function complementary products that can be provided additionally to the main meal proposition may provide a platform enabling enhanced flexibility for interventions additional to main meal products.

***Example 2: Fibre based composition for influencing the gut microbiome and gastrointestinal health***

This example describes a combination of ingredients that served to alter the microbiome and imparted physiological effects on dogs through an altered gut microbiota as measured in the faeces. This altered gut microbiota is associated with the beneficial effects seen in example 1. i.e.. Enhancement of the microbiome, was associated with reduced incidence of diarrhoea stool consistency and altered lymphocyte levels, a key while blood cell or leukocyte involved in antigen clearance and inflammation.

**Materials and Methods**

Study design and analytical methods as in Example 1

*Method*

The method involves the extraction of DNA from a freshly produced faecal sample by a means such as the QIAamp Power Faecal DNA kit (Qiagen) and subsequently the use of molecular biology techniques to detect the 16S rDNA or rRNA present or other genetic features thus determining the bacterial abundance and taxon or species richness of the microbial community in faeces or other gastrointestinal sample. After DNA extraction from freshly produced faeces and sequencing of the DNA by techniques such as 16S rDNA amplicon, shotgun, metagenome, IlluW ua, nanopore or other DNA sequencing techniques, the resulting DNA sequences are clustered to species (>98% ID) level and the relative abundance of the taxa is determined for the individual OTUs as a proportion of the total sequences. The total number of OTUs and relative abundance data is used to calculate Shannon diversity which accounts for both abundance and evenness of the species detected. Shannon Diversity can be calculated by the following method:

$$\text{Shannon Index (H)} = - \sum_{i=1}^{s} p_i \ln p_i$$

After determination of the diversity of the microbiota using functions such as alpha diversity including Shannon diversity index and total OTU numbers with the sample, diversity can be compared between samples from cohorts that are collected at the start and end of the control diet and samples from cohorts that are collected at the start and ed of the SBP/GOS/Cellulose diet.

**Microbiota Analysis**

Following bioinformatics processing, a total of 5,160 OTUs were identified, which was reduced to 404 OTUs after removal of rare sequences. After rare removals, table density (fraction of non-zero values) was at 0.752 and number of reads averaged 82,798 count per samples (min: 26,610. max: 167,912). Taxonomic assignment using the Silva database (v132, 2020) suggested classifications from the dataset fell into 3 phyla, 33 families, and 84 genera. While 158 OTUs (39%) were assigned to the species level. Bacterial alpha diversity was estimated using the Shannon index to account for both abundance and evenness of the species present within a sample. Shannon diversity (Shannon, 1997) was estimated at species level, and represented by mean +/-95% confidence intervals. In order to explore the OTU composition when the dogs received the microbiota altering ingredients a nMDS with Bray-Curtis on proportions and Phate (Moon et al.. 2019) on the logit alt. transformed data was performed.

**Results and Discussion**

**Microbial Diversity**

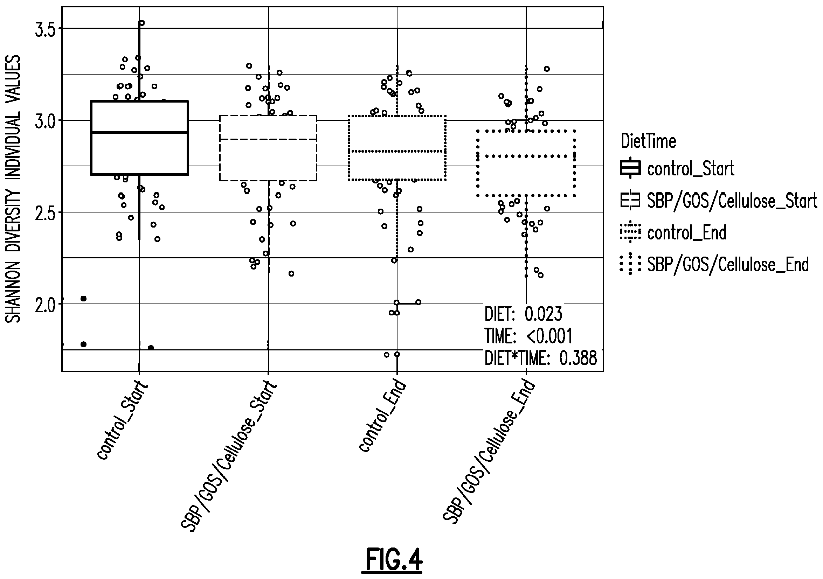

The estimated mean bacterial diversity in the faecal microbiota was lower ($p$=0.023) while the animals received the microbionic supplement ingredients compared to while they received the control diet alone as measured by the Shannon index. Statistical analysis suggested a significantly reduced diversity over time within both feeding phases, however there was no significant interaction between diet and time over which the diet was fed suggesting that changes in the microbial diversity occurred early within the feeding phase. Several outlying datapoints showed reduced diversity (SI $\leq$ 2.1) relative to the majority of the cohort while receiving the control diet over the 21 day feeding phase ( _End) yet the variation within the cohort was visibility reduced (Figure 4, control_End). This is suggestive that the variation in faecal diversity was reduced within the cohort on receipt of the diet supplement. Enhanced diversity was not observed across the cohort suggesting a generalised stabilisation effect on the microbiota of some animals during the course of the 21-day supplement feeding phase.

These data are consistent with the observed reduction in diarrhoetic faeces, enhanced stool consistency reduction in faecal pH and increased transit and defecation frequency (see Example 1; "fibre-based composition for influencing faecal pH through the gut microbiota, and gastrointestinal health and resilience").

**Bacterial Abundance within the Faecal Microbiota**

Due to the number of phylogenies within the microbiota and individual variation in the residing microbiota, consistent differences across a cohort can be challenging to identify. Mean abundance of several microbial families were significantly different when the animals received the supplement. Again, these families were not in every case altered over time within the feeding phase and the effect on the estimated group mean was observed even after a short 2-4 day feeding period. Increased abundance of OTUs attributed to the Bactcrioidaceae family associated with insoluble carbohydrate / fibre consumption, leanessness and solid faeces consistency was observed while the animals received the supplement. Meanwhile while the immunogenic Erysipelotrichaccae were significantly reduced throughout the period while the dogs received the microbiome ingredient supplement. Enhancement of immunoactive populations in the faecal microbiota of the senior cohort alongside the significantly altered blood leukocyte population (lymphocyte data see example 1) is suggestive that the dietary supplement may additionally be effective in young animals and during immune priming. The Peptostreptococcacac family associated with gastrointestinal health in humans due to their role in mucin turnover were reduced in the faeces of animals whilst receiving the supplement combination. These data warrant additional investigation as mucin metabolism by the Pcptostreptococcaeae and other members of the Phylum Firmicutes may be a rich energy source for the colonic cpthclium and a contributor to a positive energy balance in the host. As such, these data may suggest an effect on satiation and obesity.

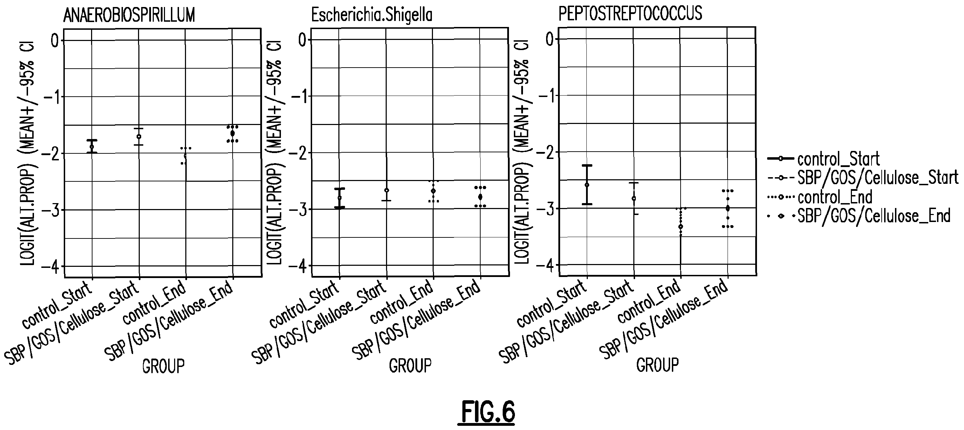

When considering microbes assigned to the *Peptostreptococcus* genus the same supplement combination was not associated with a reduced abundance of pcptostreptococci relative to the total faecal microbial community (Figure 6), however a significant interaction with time was detected with the abundance reducing over time in animals whilst receiving the control diet. This suggests that alterations in related organisms with highly similar 16S rDNA sequences may be responsible for the evenness of the microbiota throughout the phase. Of note is the closely related Mogibactcriacaeae family, which have only minor differences in the 16S rDNA biomarker sequence compared to peptostreptococci, and which are associated with enhanced energy balance and consequent fat mottling in red meat from production animals.

A further contributor to energy balance and immune function was detected in the genus *Anaerobiospirillium* where the abundance of these organisms were consistently increased in the faecal communities of animals while they were in receipt of the control diet compared to when they were fed the supplement. Bacterial species from this genus such as *Anaerobiospirillium succiniproducens* are prolific producers of succinate, a key metabolite in the control of inflammation, bacterial metabolism and an intermediary in the tricarboxylic acid (TCA) cycle. Finally, the Escherichia / Shigella microbial biomarker from the phylum Proteobacteria are recognised commensals with opportunistic pathogenic potential and are genetically identical (by analysis of the 16S rDNA biomarker sequences) to many severe gastrointestinal pathogens. Similarly, to the peptostreptococci the relative abundance of the *Escherichia / Shigella* (including *E. coli, E. fetus* and all *Shigella* subtypes) microbial community showed an interaction with time. The abundance of *Escherichia / Shigella* increased relative to the total population during the 21-day phase while the animals received the control diet, and conversely levels fell during the period the dogs received the supplement (Figure 6). These data again suggest a role for the supplement in feeding to enhance the microbiome not limited to older animals but to positively enhance and stabilise the colonic microbiota in dogs across the range of ages and particularly where diarrhoeal faeces, stool consistency, immune priming or energy balance are desirable as physiological effects.

The present disclosure may also be described by reference to the following numbered paragraph(s) "para(s)".

1. A composition suitable for administration to a companion animal comprising at least one of the ingredients selected from: galactooligosaccharides (GOS) of about 0.05 g/day to about 5.0 g/day; beet pulp and/or chicory pulp to a total amount of about 0.1 g/day to about 10 g/day; and cellulose from about 0.2 g/day to about 30.8 g/day.

2. The composition of para 1, wherein at least one of the ingredients is selected from: GOS of about 0.05g to about 3.6 g/day: beet pulp and/or chicory pulp to a total amount of about 1.65 g/day to about 9.52 g/day; and cellulose from about 5.32 g to about 25.98 g/day.

3. The composition of para 2, wherein the companion animal is a canid.

4. The composition of para 3, wherein the canid is a dog.

5. The composition of para 3 or 4 suitable for an about 5 to about 10 kg body weight canid or dog wherein at least one of the ingredients is selected from: beet pulp and/or chicory pulp to a total of about 1.65 g/day to about 5.38 g/day; cellulose of about 4.32 g/day to about 12.68 g/day and GOS of about 0.05 to about 3.6 g/day.

6. The composition of paras3 or 4 suitable for an about 10 to about 25 kg body weight canid or dog wherein at least one of the ingredients is selected from: beet pulp and/or chicory pulp to a total amount of about 2.14 g/day to about 6.36 g/day; cellulose of about 6.89 g/day to about 15.86 g/day and GOS of about 0.1 to 3.52 g/day.

7. The composition of paras 3 or 4 suitable for an about 25 kg or greater body weight canid or dog wherein at least one of the ingredients is selected from: beet pulp and/or chicory pulp to a total amount of about 3.72 g/day to about 9.52 g/day; cellulose of about 11.95 g/day to about 25.98 g/day and GOS of about 1.44 to 3.52 g/day.

8. The composition of any one of the previous paras, wherein the ratio of the ingredients is about 1: about 3.3: about 0.3 for total beet pulp and/or chicory pulp: cellulose: GOS.

9. The composition of any of the previous paras comprising at least 2 of the ingredients in the ranges specified.

10. The composition of any one of the previous paras comprising 3 of the ingredients in the ranges specified.

11. The composition of any one of the previous paras wherein at least one of the ingredients is GOS in the range specified.

12. The composition of any one of the previous paras wherein the composition is a food, supplement, nutraceutical or pharmaceutical.

13. The composition of para 12, wherein the composition is a food that is nutritionally complete and at least a portion of the food comprises dry matter.

14. Tire composition of para 13, wherein the nutrient profile of the food as a percentage of dry matter in the food comprises about 7.5% to about 19.8 % dietary fiber, about 10.0% to about 19.8% crude fat, about 2.5% to about 44.0% protein, and about 0.08% to about 4.4% indigestible protein.

15. The composition of para 12, wherein the composition is a supplement or nutraceutical and at least a portion of the supplement or nutraceutical comprises dry matter.

16. The composition of para 13, wherein the nutrient profile of the food as a percentage of the dry matter in the composition is about 0.9% to about 99.9% dietary fiber, about 1.0% to about 19.8% crude fat, about 2.5% to about 44.0% protein, and about 0.08% to about 4.4% indigestible protein.

17. The composition of the any one of the previous paras for use in a medicament.

18. The composition of any one of the previous paras for use in treating soft stools, loose faeces, gastro-intestinal dysbiosis or enhancing gut microbiome resilience in a companion animal.

19. The composition of any one of the previous paras for use in changing the fecal pH of a companion animal.

20. The composition of para 19 for use in decreasing the fecal pH of a companion animal.

21. The composition of any one of the previous claims for use in altering the microbiome in the companion animal.

22. A method of changing the microbiome of a companion animal by administering the composition of any one of paras 1 to 16 to a companion animal.

23. The method of para 22, wherein the microbiome is the gastro-intestinal microbiome of the companion animal.

24. The method of any one of paras 22 to 23, wherein the animal is a senior or geriatric animal.

25. The method of para 22, further comprising a first step of determining the health of the companion animal's microbiome, and the composition is administered to the companion animal when there is a determination of a healthy or an unhealthy microbiome detected in the first step, preferably an unhealthy microbiome.

26. The method of para 23, wherein the first step comprises detecting at least one, two, preferably at least three or at least four bacterial taxa in a sample obtained from the companion animal; wherein the presence of at least one, two, preferably at least three or at least four bacterial taxa is indicative of a healthy microbiome.

27. The method of any one of paras 22 to 26. wherein the companion animal is a canid.

28. The method of para 27, wherein the bacterial taxa are bacterial families selected from the group consisting of Bactcriodacaeae and Fusobacteriaceae.

29. The method of para 27, wherein the bacterial taxa is the Anaerobiospirillum genus.

30. The method of para 25, wherein the first step comprises detecting at least one, two, preferably at least three or at least four bacterial taxa in a sample obtained from a companion animal, wherein the presence of at least one, two, preferably at least three or at least four bacterial taxa is indicative of an unhealthy microbiome.

31. The method of para 30, wherein the companion animal is a canid.

12. The method of para 3 J, wherein the bacterial taxa are bacterial families selected from Erysipelotrichaceae and Peptostreptococcacea.

33. The method of para 25, wherein the first step comprises quantitating one or more bacterial families in a sample obtained from the companion animal to determine their abundance; and comparing the abundance to the abundance of the same families in a control data set: wherein an increase or decrease in the abundance of the one or more bacterial families relative to the control data set is indicative of an unhealthy m icrobiome.

34. The method of para 33, wherein the families are selected from the group of Bacteroidaceae, Erysipelotrichacae, Fusobacteriaceae and Peptostreptococceae.

35. The method of any one of paras 33 to 34, wherein the control data set comprises microbiome data of a companion animal at the same life stage or a life stage younger than the biological age of the companion animal.

36. The method of any one of paras 33 to 35. wherein the companion animal is a canid.

37. The method of any one of para 35. wherein the companion animal is a puppy or kitten, adult, senior or geriatric.

38. The method of any one of paras 22 to 37, further comprising a step of changing the microbiome composition of the companion animal of any one of paras 25 to 26, 30, 33 to 35, 37 or the microbiome composition of the canid of any one of paras 27 to 29, 31 to 32 and 36.

39. The method of para 25, wherein the first step comprises calculating the diversity index for the number of species within the companion animal's microbiome and comparing the diversity index to the diversity index of a control data set.

40. The method of para 39, wherein the companion animal is a canid.

41. The method of any one of paras 25 to 40. wherein the first step comprises determining the health of the companion animal's microbiome in any one of paras 25 to 26, 30, 33 to 35, 37, 38, 39, or the health of the canid's microbiome in any one of paras 27 to 29, 31 to 32, 36. 38 and 40, by the method of any one of paras 25 to 40, on at least two time points.

42. The method of para 41, wherein the two time points are at least 1 week, 2 weeks, 1 month, 2 months, 3 months, 4 months, 5 months or 6 months apart.

43. The method of anyone of paras 26 to 42. wherein the sample is from the gastrointestinal tract.

44. The method of para 43, wherein the sample is a faccal sample, an ilcal sample, a jejunal sample, a duodenal sample or a colonic sample.

45. The method of claim and one of paras 2 5 to 45, wherein the health of the microbiome is determined before and after administration of the composition.

46. The method of any one of paras 26 to 45, wherein the bacteria taxa of any one of 26 to 31 and 38, the bacterial families of any one of paras 33 to 38, the bacterial species of paras 39 to 40 is detected by means of DNA sequencing, RNA sequencing, protein sequence homology or another biological marker indicative of the bacterial species.

47. The method of anyone of paras 27 to 29, 31 to 32, 36 to 38. 40. 41 to 46. wherein the canid is a dog.

48. The composition of any one of paras 1 to 21 for use to increase the numbers of bacteria in any of the Bactcrioidaceae and/or Fusobacteriaceae families present in the gastro-intestinal tract or faeces of a companion animal compared to the number of said bacteria present in the companion animal before administration of the diet and/ or for reducing the numbers of bacteria in any of the Erysipelotrichaccae and/or Peptostreptococcaceae families.

49. A method for increasing the bacterial counts of at least one of the bacterial taxa in a group consisting of Bacteriodaceae and/or Fusobacteriaceae, in the gastro-intestine and/or faeces of a companion animal by administering the composition of any one of paras 1 to 21 and/ or for reducing at least one of the bacteria in a group consisting of the numbers of Erysipelotrichaccae and/or Peptostreptococcaceae families.

50. The composition of any one of paras 1 to 21 for use to decrease the number of Erysipelotrichaccae and/or Peptostreptococcaccac.

51. The composition of any one of paras 1 to 21 for use in increasing the number of bacterial counts of at least one of the bacterial taxa in a group comprising Bacteriodaceae and Fusobacteriaceae in the microbiome of a companion animal.

51. The composition of any one of paras 1 to 21 for use in reducing or stabilising the diversity of the microbiome

53 The composition of any one of paras 1 to 21 for use in weight maintenance and/or increasing satiety in a companion animal.

54. The composition of paras 41 to 48, 51 to 52, wherein the companion animal is a canid.

55. The composition of para 53, wherein the canid is a puppy, adult, senior or geriatric canid, preferably where the canid is a dog.

**Claims**

1. A composition for use in treating soft stools, loose faeces, gastro-intestinal dysbiosis or enhancing gut microbiome resilience in a companion animal by administration of galactooligosaccharides (GOS) of 0.05 g/day to 5.0 g/day; beet pulp and/or chicory pulp to a total amount of 0.1 g/day to 10 g/day; and cellulose from 0.2 g/day to 30.8 g/day, , and wherein the ratio of the ingredients is about 1: about 3.3: about 0.3 for total beet pulp and/or chicory pulp: cellulose: GOS, wherein the composition is further for use in increasing the numbers of bacteria in any of the Bacterioidaceae and/or Fusobacteriaceae families present in the gastro-intestinal tract or faeces of a companion animal compared to the number of said bacteria present in the companion animal before administration of the composition and/or for use in reducing the numbers of bacteria in any of the Erysipelotrichaceae and/or Peptostreptococcaceae families.

2. The composition for the use of claim 1, by administration of GOS of 0.05g to 3.6 g/day; beet pulp and/or chicory pulp to a total amount of 1.65 g/day to 9.52 g/day; and cellulose from 5.32 g to 25.98 g/day.

3. The composition for the use of any preceding claim, wherein the companion animal is a canid

4. The composition for the use of any preceding claim, wherein the canid is a dog.

5. The composition for the use of any one of claims 3 or 4, by administration of beet pulp and/or chicory pulp to a total of 1.65 g/day to 5.38 g/day; cellulose of 4.32 g/day to 12.68 g/day and GOS of 0.05 to 3.6 g/day, wherein the composition is suitable for a 5 to 10 kg body weight canid or dog.

6. The composition for the use of any one of claims 3 or 4, by administration of beet pulp and/or chicory pulp to a total of 2.14 g/day to 6.36 g/day; cellulose of 6.89 g/day to 15.86 g/day and GOS of 0.1 to 3.52 g/day, wherein the composition is suitable for a 10 to 25 kg body weight canid or dog.

7. The composition for the use of any one of claims 3 or 4, by administration of beet pulp and/or chicory pulp to a total of 3.72 g/day to 9.52 g/day; cellulose of 11.95 g/day to 25.98 g/day and GOS of 1.44 to 3.52 g/day, wherein the composition is suitable for an 25kg or greater body weight canid or dog.

8. The composition for the use of any previous claims wherein the composition is a food, supplement, nutraceutical or pharmaceutical.

9. The composition for the use of claim 8, wherein the composition is a food that is nutritionally complete and at least a portion of the food comprises dry matter.

10. The composition fore the use of claim 9, wherein the nutrient profile of the food as a percentage of dry matter in the food comprises 7.5% to 19.8 % dietary fiber, 10.0% to 19.8% crude fat, 2.5% to 44.0% protein, and 0.08% to 4.4% indigestible protein.

11. The composition for the use of claim 8, wherein the composition is a supplement or nutraceutical and at least a portion of the supplement or nutraceutical comprises dry matter.

12. The composition for the use of claim 9, wherein the nutrient profile of the food as a percentage of the dry matter in the composition is 0.9% to 99.9% dietary fiber, 1.0% to 19.8% crude fat, 2.5% to 44.0% protein, and 0.08% to 4.4% indigestible protein.

FIG.1

**FIG.2**

EP 4 430 959 A2

FAECAL WET WEIGHT PER DOG (g)

DAILY NUMBER OF FAECES PER DOG (ALL DATA)

FIG.3

FIG.4

FIG.5

**FIG.6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020014292 W
- WO 2020150712 A